# EUROPEAN PATENT SPECIFICATION

(11) **EP 4 165 030 B1**
(45) Date of publication and mention of the grant of the patent: **29.07.2026**
(21) Application number: 21820820.5
(22) Date of filing: 08.06.2021
(51) Int. Cl.: C07D 401/04, C07C 233/65, A01N 43/56, A01P 7/00

(54) **THE PROCESS OF PREPARATION OF ANTHRANILAMIDES**
VERFAHREN ZUR HERSTELLUNG VON ANTHRANILAMIDEN
PROCÉDÉ DE PRÉPARATION D'ANTHRANILAMIDES

(30) Priority: 08.06.2020 US 202063035865 P
(43) Date of publication of application: 19.04.2023
(73) Proprietor: Adama Makhteshim Ltd., 8410001 Beer-Sheva (IL)
(72) Inventor: SUEZ, Gal, 8619006 Dimona (IL); FORCKOSH, Hagit, 8479154 Beer Sheva (IL); GOLUB, Yanai, 8496501 Omer (IL); LIE, Jie, Nanjing, Jiangsu 210046 (CN); CHEN, Bob, Shanghai 201199 (CN); PARNES, Regev, 8531500 Kibbutz Mishmar Hanegev (IL)
(74) Representative: Modiano, Gabriella Diana
(86) International application number: PCT/CN2021/098904
(87) International publication number: WO 2021/249396

(56) References cited:
- WO-A1-2009/121288
- WO-A1-2019/207595
- CN-A- 102 020 633
- CN-A- 110 028 489
- CN-A- 112 552 284
- YANG GUI-QIU, ZHANG YU, YANG HUI-BIN, WU HONG-FEI, YU HAI-BO, LI BIN: "New Method for Synthesizing Cyantraniliprole", MODERN AGROCHEMICALS, vol. 11, no. 1, 29 February 2012 (2012-02-29), CN , pages 22 - 24,29, XP009532763, ISSN: 1671-5284, DOI: 10.3969/j.issn.1671-5284.2012.01.005

## Description

### FIELD OF THE INVENTION:

This invention relates to the process of preparation of arthropocidal anthranilamides by reaction of acyl chloride and the corresponding anthranilic acid amides wherein the process is carried out in the absence of acid scavenger.

### BACKGROUND OF THE INVENTION:

Arthropodicidal anthranilamides have wide range of applications and are previously disclosed as active pesticides in PCT patent applications such as WO 2001/070671, WO 2003/015519, WO 2003/015518 and WO 2004/067528. General methods of their preparation are disclosed for example in PCT Patent Publications WO 2001/070671, WO 2006/062978, WO 2003/016283, WO 2004/011447 and WO 2003/015519.

It has already been disclosed that anthranilamides of formula I(a) and I(b) could be obtained by reacting the corresponding anthranilic acids or amides of anthranilic acids with heteroaryl carboxylic acids or their acyl halides in the presence of acid scavengers, usually, amines as disclosed in WO 2006/062978, WO 2019/207595 and WO 2021/033172. However, these preparative methods still must be improved for safe economic commercial operation. In particular, the acid scavengers used in the previously recited processes belong to organic amines class and should be present in molar excess toward the starting pyrazole carboxylic acid. The resulting hydrochloride salts of said acid scavengers are hardly removed from the process and their presence in most cases leads to the formation of "cake" which cannot be easily removed from the reaction vessel. In addition, the presence of organic base as an acid scavenger in the aforementioned processes, affects the cyano group in the anthranilamides containing cyano-substituents and reduces the yield and purity of final anthranilamide. It would be highly desirable to have an improved process for the production of the anthranilamides of formula (Ia) and/or (I(b) which is suitable for industrial use, highly efficient, low-cost, environmentally friendly, and provides a high yield in a short reaction time, thereby overcoming the deficiencies of the prior art. The present subject matter provides such a process.

### SUMMARY OF THE INVENTION:

The present invention provides a process of preparation of anthranilamides of formula (Ia) or (Ib) 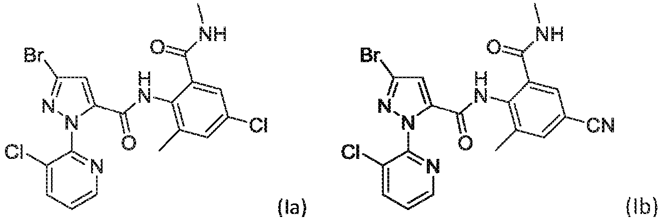 comprising reaction of compound of formula (II) 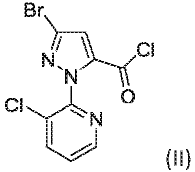 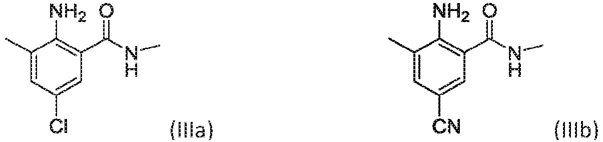 with the compound of formula (Illa) or (IIIb) in the presence of aromatic solvent wherein the hydrochloric acid formed is distilled out from the reaction mixture during the process and the process is carried out in the absence of acid scavenger, which is selected from organic amines.

### DETAILED DESCRIPTION OF THE INVENTION:

### Definitions:

Prior to setting forth the present subject matter in detail, it may be helpful to provide definitions of certain terms to be used herein. Unless defined otherwise, all technical and scientific terms used herein have the same meaning as is commonly understood by one of skill in the art to which this subject matter pertains.

The term "a" or "an" as used herein includes the singular and the plural, *unless specifically stated* otherwise. Therefore, the terms "a," "an," or "at least one" can be used interchangeably in this application.

Throughout the application, descriptions of various embodiments use the term "comprising"; however, it will be understood by one skilled in the art, that in some specific instances, an embodiment can alternatively be described using the language "consisting essentially of" or "consisting of".

For purposes of better understanding the present teachings and in no way limiting the scope of the teachings, unless otherwise indicated, all numbers expressing quaritities, percentages, or proportions, and other numerical values used in the specification and claims, are to be understood as being modified in all instances by the term "about."

Accordingly, unless indicated to the contrary, the numerical parameters set forth in the following specification and attached claims are approximations that may vary depending upon the desired properties sought to be obtained. At the very least, each numerical parameter should at least be construed in light of the number of reported significant digits and by applying ordinary rounding techniques. In this regard, use of the term "about" herein specifically includes ±10% from the indicated values in the range. In addition, the endpoints of all ranges directed to the same component or property herein are inclusive of the endpoints, are independently combinable, and include all intermediate points and ranges.

Certain compounds of this invention can exist as one or more stereoisomers. The various stereoisomers include enantiomers, diastereomers, and geometric isomers. One skilled in the art will appreciate that one stereoisomer may be more active and/or may exhibit beneficial effects when enriched relative to the other stereoisomer(s) or when separated from the other stereoisomer(s). Additionally, the skilled artisan knows how to separate, enrich, and/or to selectively prepare said stereoisomers.

Accordingly, the compounds of the invention may be present as a mixture of stereoisomers, individual stereoisomers, or as an optically active form.

The present invention provides technology useful for the successful and convenient preparation of anthranilic acid amides in the absence of acid scavenger. Moreover, said technology includes distillation out of the hydrochloric acid formed during the preparation of anthranilic acid amides
According to an embodiment, the present invention concerns the process of preparation of anthranilamides of formula (Ia) 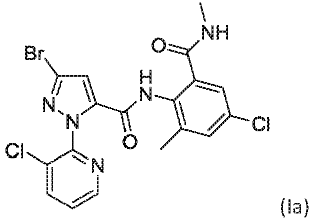 comprising reaction of compound of formula (II) 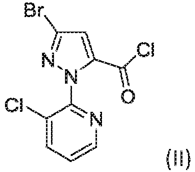 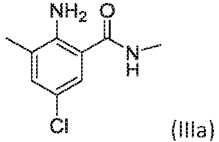 with the compound of formula (IIIa) in the presence of aromatic solvent, wherein the hydrochloric acid formed is distilled out from the reaction mixture during the process and the process is carried out in the absence of acid scavenger, which is selected from organic amines.

In purpose of the invention the acid scavenger is referred to a chemical substance added to a reaction mixture in order to remove or de-activate acids generated in the process, for example, organic amines can serve as acid scavengers.

According to another embodiment, the present invention concerns the process of preparation of anthranilamides of formula (Ib) 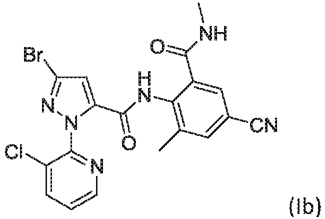 comprising reaction of compound of formula (II) 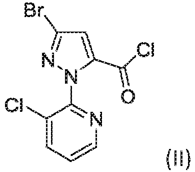 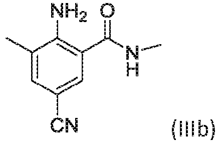 with the compound of formula (IIIb) in the presence of aromatic solvent, wherein the hydrochloric acid formed is distilled out from the reaction mixture during the process and the process is carried out in the absence of acid scavenger, which is selected from organic amines.

Pyrazolecarboxylic acid chloride of formula (II) can be prepared from the corresponding pyrazole carboxylic acids using known methods, for example by reaction of said carboxylic acids with chlorination agents like thionyl chloride, oxalyl chloride and the like as recited in WO 2001/070671, WO 2003/015519, WO 2003/015518, WO 2003/016283. The corresponding starting pyrazole carboxylic acid can be prepared using methods described in literature, for example in WO 2003/015519, WO 2003/016283, WO 2006/102025, WO 2013/076092.

Anthranilic acid amides (Illa) and (IIIb) can be prepared according to the methods described for example in WO2003/016300, WO 2008/010897, WO 2013/007603.

In some embodiment, the process carried out wherein the molar ratio of compound of formula (II) to the compound of formula (IIIa) is about 1.2:1 to about 1:1.2.

In some another embodiment, the process carried out wherein the molar ratio of compound of formula (II) to the compound of formula (IIIb} is about 1.2:1 to about 1:1.

In some embodiment, the process carried out "in the absence of acid scavenger" refers to the process including not more than 0.01 mole of acid scavenger on each 1 mole of pyrazole carboxylic acid of formula (II).

In some embodiment, the process carried out "in the absence of acid scavenger" refers to the process including not more than 0.05 mole of acid scavenger on each 1 mole of pyrazole carboxylic acid of formula (II).

In some embodiment, the process carried out "in the absence of acid scavenger" refers to the process including not more than 0.1 mole of acid scavenger on each 1 mole of pyrazole carboxylic acid of formula (II).

In another embodiment, the process carried out "in the absence of acid scavenger" refers to the process including not more than 0.5 mole of acid scavenger on each 1 mole of pyrazole carboxylic acid of formula (II).

For the purpose of the present invention, the acid scavenger refers to the chemical compound added to the reaction mixture which is able to remove or de-activate impurities and unwanted reaction products, in particular, hydrochloric acid formed during the process of preparation of anthranilamides of formula (Ia) and (Ib).

In some embodiments, the acid scavengers are referred to organic bases such as amines; e.g triethylamine, diethylamine, substituted or unsubstituted pyridine, picoline, lutidine and the like.

The process is suitably carried out in the aromatic solvent such as toluene, xylene, ethylbenzene, chlorobenzene and the mixtures thereof. Optionally, additional inert solvent could be used in the inventive process.

According to another embodiment the hydrochloric acid formed in the process is distilled out from the reaction mixture during the process.

In a class of this embodiment is the process wherein a distillation of hydrochloric acid is performed at a temperature in the range of from 75° C to 100° C.

In a class of this embodiment is the process wherein a distillation of hydrochloric acid is performed at a temperature in the range of from 75° C to 85° C.

In a class of this embodiment is the process wherein a distillation of hydrochloric acid is performed at a temperature in the range of from 85° C to 100° C.

In a class of this embodiment is the process wherein a distillation of hydrochloric acid is performed at a temperature of 80 °C.

In a class of this embodiment is the process wherein a distillation of hydrochloric acid is performed at a temperature of 85 °C.

In yet another embodiment, the distillation of hydrochloric acid is performed at a temperature of 90° C to 100 °C.

In a class of this embodiment is the process wherein a distillation of hydrochloric acid is performed at a pressure of 380 to 420 mm Hg.

In another embodiment the process of distillation of hydrochloric acid is performed at a pressure of 420 to 450 mm Hg.

In a class of this embodiment is the process wherein a distillation of hydrochloric acid is performed at a pressure of 380 to 400 mm Hg.

Without further elaboration, it is believed that one skilled in the art using the preceding description is suitable to utilize the present invention to its fullest extent. The following Examples are, therefore, to be construed as merely illustrative, and not limiting of the disclosure in any way whatsoever.

### EXAMPLES:

### EXAMPLE 1: Preparation of cyanthraniliprole (Ib):

a) Preparation of 3-bromo-1-(3-chloropyridin-2-yl)-1*H*-pyrazole-5-carbonyl chloride (compound (II)): 3-bromo-1-(3-chloropyridin-2-yl)-1*H*-pyrazole-5-carboxylic acid (40 kg, 132 moles) and toluene (97 kg) were charged into a reactor and the resulting suspension was stirred and heated to 80°C. Thionyl chloride (20 kg, 168 moles) was added dropwise during 120 minutes to the above suspension. Then the resulting mixture was stirred for 1 hr. After reaction completion by HPLC, solvent was distilled off at 80°C under reduced pressure of 450 mbar and 66 kg of toluene was added. After that, the final crude mixture was filtered and washed with 5-10 kg of toluene to obtain 3-bromo-1-(3-chloropyridin-2-yl)-1*H*-pyrazole-5-carbonyl chloride as 27% w/w toluene solution.
b) In another reactor, 2-amino-5-cyano-*N*,3-dimethylbenzamide (20g, 102 mmol) was charged in 150 gr toluene and azeotropic distillation was done to remove water. Then, the resulting solution was heated to 75°C under vacuum of 420 mbar and the solution of 3-bromo-1-(3-chloropyridin-2-yl)-1*H*-pyrazole-5-carbonyl chloride prepared in a) was added dropwise to the 2-amino-5-cyano-*N*,3-dimethylbenzamide in toluene under vacuum. Then the reaction mass was stirred for 2h. During the stirring, fresh toluene is added (380 gr) under vacuum. After reaction completion by HPLC, the precipitated solid is filtered. The resulting solid was dried overnight under vacuum at 60°C to afford 46.4g cyantraniliprole of formula (la) as light brown solid (yield- 94.8%), purity by HPLC- 98.8%.

### EXAMPLE 2: Comparative example for the preparation of cyantraniliprole (Ib) in the presence of 3-picoline as acid scavenger:

2-amino-5-cyano-*N*,3-dimethylbenzamide (20 g, 1.02 mmol) and toluene (193 gr) were mixed and heated to 80°C under vacuum of 400 mbar. Then the solution of 3-bromo-1-(3-chloropyridin-2-yl)-1*H*-pyrazole-5-carbonyl chloride in toluene (115.6 gr, 98 mmol) prepared in Example 1 a) was added dropwise to the reaction mass during 120 minutes while vacuum distillation occurred. After that vacuum was removed, 3-picoline was added (2.4 gr, 26 mmol) and the reaction mixture was stirred for 6h, then the reaction mixture is cooled to ambient temperature and the solid is filtered and washed with toluene and dried under vacuum at 70°C overnight to afford cyantraniliprole of formula (Ib) as 28.2g (51% yield) as light brown solid with purity by HPLC 84.4%.

### EXAMPLE 3: Preparation of chlorantraniliprole (Ia)

2-amino-5-chloro-N,3-dimethylbenzamide (22g, 0.111 mol mol) was charged in 220 gr toluene and the mixture has been heated up to 85 °C under pressure of 450 mbar. The solution of 3-bromo-1-(3-chloropyridin-2-yl)-1H-pyrazole-5-carbonyl chloride in toluene (0.107 mol, 27% w/v) prepared in Example 1 a) has been added dropwise was added dropwise to the reaction mass during 120 minutes, while vacuum distillation occurred. Then 50 gr of toluene was added dropwise and distillation continued for additional one hour. After that 3 gr (0.038 mol) of pyridine added dropwise to the reaction mixture and the reaction cooled to r.t. The crude product was filtered, washed with toluene and then with methanol and dried to afford chloraritraniliprole of formula (Ia) as 46.5 gr (90% yield) as off-white solid with purity by HPLC 98.0 %.

## Claims

1. A process of preparation of anthranilamides of formula (Ia) or (Ib) 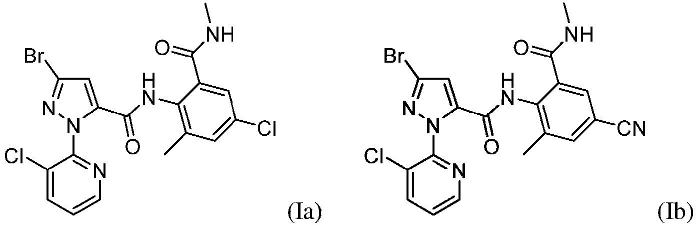 comprising reaction of compound of formula (II) 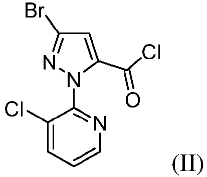 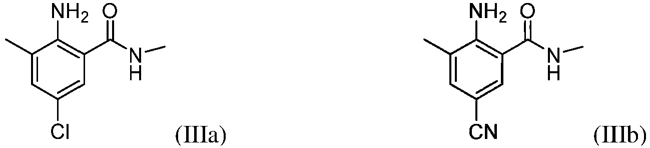 with the compound of formula (IIIa) or (IIIb) in the presence of aromatic solvent wherein the hydrochloric acid formed is distilled out from the reaction mixture during the process and the process is carried out in the absence of acid scavenger, which is selected from organic amines.

2. The process according to claim 1, wherein the organic amines are selected from triethylamine, diethylamine, substituted or unsubstituted pyridine, picoline and lutidine.

3. The process according to any of claims 1-2 wherein the aromatic solvent is selected from the group consisting of toluene, xylene, ethylbenzene, chlorobenzene and the mixtures thereof.

4. The process according to claim 1 wherein distillation of hydrochloric acid is performed at a temperature in the range of from 75° to 100° C and at a pressure of 380 to 450 mm Hg (50.7 to 60.0 kPa).

5. The process according to claim 4 wherein distillation of hydrochloric acid is performed at a temperature in the range of from 75° to 85° C.

6. The process according to claim 4 wherein distillation of hydrochloric acid is performed at a pressure of 420 to 450 mm Hg 56.0 to 60.0 kPa).

## Patentansprüche

1. Ein Verfahren zur Herstellung von Anthranilamiden mit der Formel (Ia) oder (Ib), 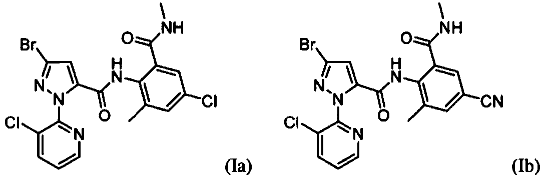 das die Reaktion einer Verbindung mit der Formel (II) 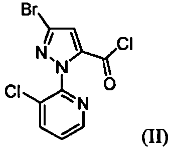 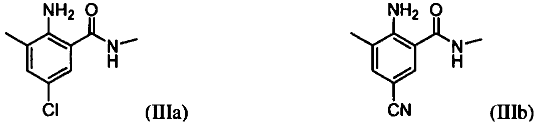 mit der Verbindung mit der Formel (IIIa) oder (IIIb) in Anwesenheit eines aromatischen Lösungsmittels umfasst, wobei die gebildete Salzsäure aus der Reaktionsmischung während des Verfahrens herausdestilliert wird und das Verfahren in Abwesenheit eines Säurefängers durchgeführt wird, der aus organischen Aminen gewählt ist.

2. Das Verfahren gemäß Anspruch 1, worin die organischen Amine gewählt sind aus Triethylamin, Diethylamin, substituiertem oder unsubstituiertem Pyridin, Picolin und Lutidin.

3. Das Verfahren gemäß einem beliebigen der Ansprüche 1-2, worin das aromatische Lösungsmittel gewählt ist aus der Gruppe bestehend aus Toluol, Xylol, Ethylbenzol, Chlorbenzol und den Mischungen davon.

4. Das Verfahren gemäß Anspruch 1, worin die Destillation von Salzsäure bei einer Temperatur im Bereich von 75° bis 100°C und einem Druck von 380 bis 450 mm Hg (50,7 bis 60,0 kPa) durchgeführt wird.

5. Das Verfahren gemäß Anspruch 4, worin die Destillation von Salzsäure bei einer Temperatur im Bereich von 75° bis 85°C durchgeführt wird.

6. Das Verfahren gemäß Anspruch 4, worin die Destillation von Salzsäure bei einem Druck von 420 bis 450 mm Hg (56,0 bis 60,0 kPa) durchgeführt wird.

## Revendications

1. Procédé de préparation d'anthranilamides de formule (Ia) ou (Ib) 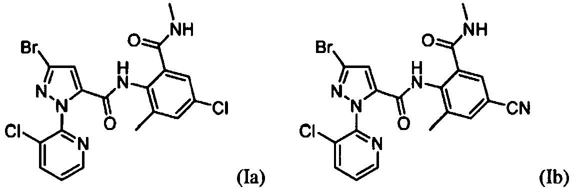 comprenant la réaction d'un composé de formule (II) 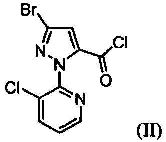 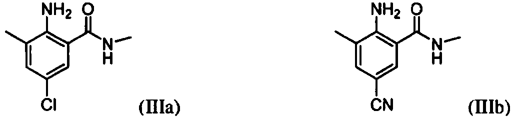 avec le composé de formule (IIIa) ou (IIIb) en présence d'un solvant aromatique, où l'acide chlorhydrique formé est éliminé par distillation du mélange de réaction au cours du procédé et le procédé est mis en œuvre en l'absence d'un agent de piégeage d'acide, qui est choisi parmi les amines organiques.

2. Procédé selon la revendication 1, où les amines organiques sont choisies parmi la triéthylamine, la diéthylamine, la pyridine substituée ou non substituée, la picoline et la lutidine.

3. Procédé selon quelconque des revendications 1 à 2, où le solvant aromatique est choisi dans le groupe constitué par toluène, xylène, éthylbenzène, chlorobenzène et leurs mélanges.

4. Procédé selon la revendication 1, où la distillation de l'acide chlorhydrique est effectuée à une température dans la plage allant de 75 °C à 100 °C et à une pression de 380 à 450 mm Hg (50,7 à 60,0 kPa).

5. Procédé selon la revendication 4, où la distillation de l'acide chlorhydrique est effectuée à une température dans la plage allant de 75 °C à 85 °C.

6. Procédé selon la revendication 4, où la distillation de l'acide chlorhydrique est effectuée à une pression dans la plage allant de 420 à 450 mm Hg (56,0 à 60,0 kPa).
